# EUROPEAN PATENT SPECIFICATION

(11) **EP 2 517 741 B1**
(45) Date of publication and mention of the grant of the patent: **13.04.2016**
(21) Application number: 12165820.7
(22) Date of filing: 26.04.2012
(51) Int. Cl.: A61M 1/14, A61M 1/36

(54) **An assembly for monitoring a cassette of a dialysis machine**
Anordnung zur Überwachung einer Kassette einer Dialysemaschine
Ensemble de surveillance d'une cassette d'une machine de dialyse

(30) Priority: 26.04.2011 IT BO20110224
(43) Date of publication of application: 31.10.2012
(73) Proprietor: Bellco S.r.l., Mirandola (IT)
(72) Inventor: Cianciavicchia, Domenico, 64040 Cavuccio (IT); Fiorenzi, Andrea, 41013 Castelfranco Emilia (IT)
(74) Representative: Bergadano, Mirko

(56) References cited:
- EP-A2- 0 771 569
- DE-C1- 19 817 995
- US-A- 4 464 172
- US-A1- 2007 012 623
- US-A1- 2007 287 959
- US-A1- 2010 089 807
- US-A1- 2011 051 136

## Description

The present invention relates to an assembly for monitoring a cassette of a dialysis machine.

Dialysis machines generally comprise a cassette having the function of ensuring a certain order in arrangement of the tubes responsible for the transport of the different liquids involved in the dialysis treatment.

Cassettes are components of the dialysis machine separated physically from the main structure of the machine itself and, in use, are fitted to a front panel of the main structure itself. Generally, cassettes are made of rigid and transparent plastic material, such as PVC.

The tubes relating to the venous line, to the arterial line and other liquid lines involved in the dialysis treatment, such as plasma or plasma water, converge in the cassette.

In this regard, cassettes can be divided internally into a plurality of fluid-tight compartments separated from one another and each of which is arranged to receive a particular fluid of the dialysis treatment.

As will be evident to those skilled in the art, it is extremely important for the level of the liquids in the various compartments of the cassette to be constantly monitored during dialysis treatment.

Currently, this monitoring is implemented by optical sensors composed of a LED and of a light receiver arranged on opposite parts of the cassette, at a height thereof at which the presence of liquid must be checked. The light detected by the receiver is closely linked to the absorbance of the medium through which the light passes. From this detection, it is possible to recognise whether or not liquid is present at the level at which the light passes through the cassette.

A measurement of this kind suffers from the drawback of only providing information regarding the presence of liquid at a specific height of the cassette, without being able to provide general information regarding the general situation of the liquid in the cassette, such as its effective level, and its state, such as its turbidity or its foaminess.

Moreover, the cassettes described above can be of different types according to the type of dialysis treatment that the dialysis machine must perform. It must be mentioned that different treatments naturally imply the use of different disposable kits, and that it is therefore extremely important to be sure that the correct cassette and the correct disposable kit has been fitted to the machine set for a specific treatment.

Currently, checking that the correct cassette has been used is left solely to the care of personnel, with the drawbacks and uncertainties that this can represent.

US 2010/0089807 discloses a dialysis machine with optical level sensors arranged to ensure that the fluid level inside a bubble trap of a cassette is above a lower level and below an upper level. DE 19817995 discloses a system for monitoring the fluid level inside a blood reservoir, the system comprising a CCD sensor.

There is the need to provide a type of sensor which is able to ensure greater efficiency in monitoring liquids inside the cassette.

A further need is that of providing a control system to check in an efficient manner the correct relation between cassette, disposable kit and type of treatment set on the machine.

The subject of the present invention is an assembly for monitoring a cassette, the essential characteristics of which are set forth in claim 1, and the preferred and/or auxiliary characteristics of which are set forth in claims 2-4.

A further subject of the present invention is a dialysis machine comprising a vision sensor useful to the monitoring assembly forming the subject of the present invention.

Yet another subject of the present invention is a cassette for dialysis machines useful to the assembly forming the subject of the present invention.

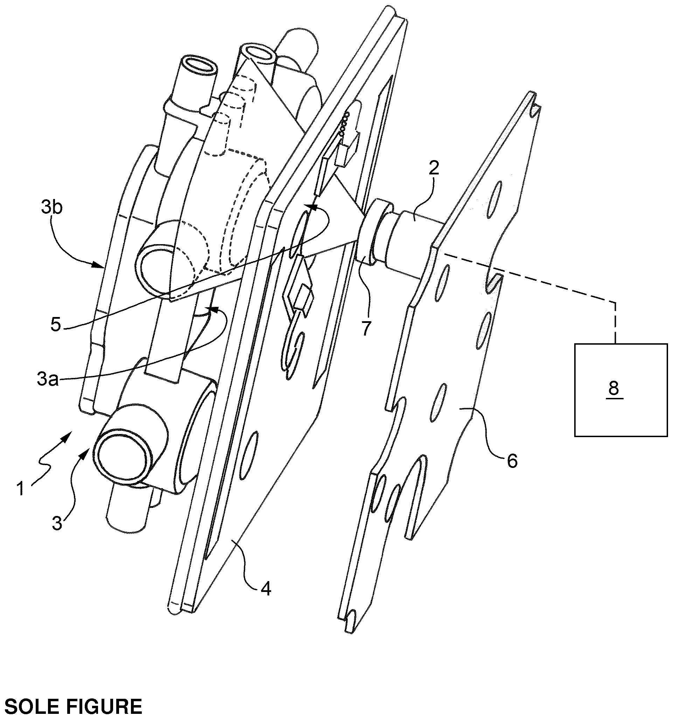

For a better understanding of the invention there is set forth below an embodiment provided purely by way of non-limiting example with the aid of the figure of the accompanying drawing, which shows an assembly constituted by a cassette and by a vision sensor as arranged in a dialysis machine.

In the figure, the reference numeral 1 indicates as a whole an assembly constituted by a vision sensor 2 and by a cassette 3 for a dialysis machine, the parts of which are not illustrated and described for simplicity. The cassette 3 is fitted to a wall 4 of the dialysis machine according to prior art solutions. An opening 5 is obtained in the wall 4, in the area of the cassette which must be analysed by the vision sensor 2.

As illustrated in the figure, the vision sensor 2 is arranged on the opposite part of the wall 4 with respect to the cassette 3 and is fitted to an internal support plate 6 of the dialysis machine.

The vision sensor 2 comprises a wide-angle lens 7 such as to allow detection of a significant area of the cassette and facing the opening 5. This significant area must necessarily comprise a plate, preferably realized with a bar code, identifying the cassette and the compartment in which it is necessary to monitor the level of liquid.

In particular, the significant area comprises the upper portion of the compartment of interest and a lower area of a vent tube that extends vertically from the upper area of the compartment of interest.

In general, the vision sensor 2 is adapted to detect images within a spectrum of wavelengths ranging from infrared to ultraviolet, preferably in the visible range.

In particular, the vision sensor of the specific example operates with a CCD technology and has a resolution of 640x480 pixels.

The vision sensor 2 is connected to a control unit 8 illustrated schematically, in which data management and processing software reads the results coming from the vision sensor 2 and converts them into actions or into alarms, according to settings entered previously.

The cassette 3 comprises a first wall 3a, which is transparent and, in use, faces the wall 4 and a second wall 3b, which is opaque/white. In this way, it is possible to obtain better contrast between the part of the cassette occupied by the liquid and the part of the cassette without liquid so as to better discriminate the level.

One or more markers are produced on the wall 3a, which have the function of reference points for visual analysis and processing of the software.

To make the visual analysis independent from the condition of the ambient light, on the wall 4 on opposite sides of the opening 5 there are housed two small white light LED illuminators, facing the wall 3a of the cassette 3. The presence of the LED illuminators also has the advantage of discriminating the film of liquid deposited on the walls of the cassette from the effective filling thereof.

The vision sensor 2 is able to provide an overall situation of the situation of the liquid inside the cassette 3, in this way managing to transmit to the control unit 8 a plurality of data relating both to the level of the liquid and to its state, in such a manner that the control unit 8 itself can, on the basis of the settings provided, take action with solutions to restore the optimal situation or with alarm signals.

In particular, with the vision sensor 2 it is possible to check both the effective level of the liquid in the compartment (therefore, not only whether the liquid is above or below a given threshold), and its physical state, which can mean, for example, its level of foaminess and its limpidity (which for blood can also mean the formation of clots).

As mentioned above, the vision sensor 2 is also able to read the code of the cassette and transmit the information to the control unit 8 which will be able to check compatibility between the cassette and the type of treatment set on the machine, and the disposable kit fitted.

To summarise, the solution in which a dialysis machine has a vision sensor to monitor the liquid inside a cassette allows, with respect to prior art, checking of the effective level of the liquid, the state thereof and that the correct type of cassette is fitted.

## Claims

1. A dialysis machine comprising a vision sensor (2), which is configured to monitor the level and the physical state of a fluid present inside a cassette (3) which is fitted to a wall (4) of the dialysis machine; said machine being **characterised in that** said vision sensor (2) is fitted to an internal support plate (6) of the dialysis machine and faces an opening (5), which is obtained on the wall (4) in the area of the cassette (3) to be monitored, and **in that** said vision sensor (2) operates with a CCD technology adapted to detect images within a spectrum of wavelengths ranging from infrared to ultraviolet and comprises a wide-angle lens and **in that** it comprises one or more illuminators, which are adapted to illuminate said cassette (3), so as to make the visual analysis independent from the condition of the ambient light; said dialysis machine comprising a control unit (8) having a data management and processing software configured to read images coming from the vision sensor (2) and to convert them into actions or into alarms according to settings entered previously; said vision sensor (2) being able to read a code of the cassette and transmit the information to the control unit (8) which is configured to check compatibility between the cassette and a type of treatment set on the machine and a disposable kit fitted.

2. A dialysis machine according to claim 1, **characterised in that** said illuminator is a white light LED illuminator.

3. A dialysis machine according to claim 1 or 2, **characterised in that** said vision sensor (2) is adapted to detect images within a spectrum of wavelengths in the visible range.

4. A dialysis machine according to any of the previous claims, **characterised in that** said vision sensor (2) has a resolution of 640x480 pixels.

## Patentansprüche

1. Eine Dialysemaschine, die einen Sichtsensor (2) aufweist, der konfiguriert ist zum Überwachen des Pegels und des physikalischen Zustandes einer Flüssigkeit, die in einer Kassette (3) vorliegt, die in eine Wand (4) der Dialysemaschine eingepasst ist; wobei die Maschine **dadurch gekennzeichnet ist, dass** sie den Sichtsensor (2) aufweist, der in eine interne Tragplatte (6) der Dialysemaschine eingepasst ist und zu einer Öffnung (5) weist, die an der Wand (4) in dem Bereich der Kassette (3), die überwacht werden soll, vorgesehen ist, und dadurch, dass der Sichtsensor (2) mit einer CCD-Technologie arbeitet, die ausgelegt ist zum Detektieren von Bildern innerhalb eines Spektrums von Wellenlängen in einem Bereich von Infrarot bis Ultraviolett und dass er eine Weitwinkellinse aufweist und dadurch dass er eine oder mehrere Leuchten aufweist, die zum Beleuchten der Kassette (3) ausgebildet sind, um die Sichtanalyse unabhängig vom Zustand des Umgebungslichtes zu machen; wobei die Dialysemaschine eine Steuereinheit (8) aufweist, die eine Daten-management- und -verarbeitungssoftware hat, die konfiguriert ist um Bilder zu lesen, die vom Sichtsensor (2) kommen und um sie in Aktionen oder in Alarme gemäß zuvor eingegebenen Einstellungen umzuwandeln; wobei der Sichtsensor (2) in der Lage ist, einen Code der Kassette zu lesen und die Information an die Steuereinheit (8) zu senden, die konfiguriert ist, um eine Kompatibilität zwischen der Kassette und einem Typ einer Behandlung, der an der Maschine eingestellt ist, und einem wegwerfbaren, eingesetzten Einsatz bzw. Zubehör zu prüfen.

2. Dialysemaschine nach Anspruch 1, **dadurch gekennzeichnet, dass** die Leuchte eine LED-Leuchte mit weißem Licht ist.

3. Dialysemaschine nach Anspruch 1 oder 2, **dadurch gekennzeichnet, dass** der Sichtsensor (2) ausgebildet ist zum Detektieren von Bildern in einem Spektrum von Wellenlängen im sichtbaren Bereich.

4. Dialysemaschine nach einem der vorhergehenden Ansprüche, **dadurch gekennzeichnet dass** der Sichtsensor (2) eine Auflösung von 640x480 Pixeln hat.

## Revendications

1. Machine de dialyse comprenant un capteur de vision (2), qui est configuré pour surveiller le niveau et l'état physique d'un fluide présent à l'intérieur d'une cassette (3) qui est montée sur une paroi (4) de la machine de dialyse ; ladite machine étant **caractérisée en ce que** ledit capteur de vision (2) est monté sur une plaque de support interne (6) de la machine de dialyse et fait face à une ouverture (5), qui est obtenue sur la paroi (4) dans la zone de la cassette (3) à surveiller, et **en ce que** ledit capteur de vision (2) fonctionne avec une technologie CCD (dispositif à couplage de charge) adaptée pour détecter des images dans un spectre de longueurs d'onde allant de l'infrarouge à l'ultraviolet et comprend un objectif grand angle et **en ce qu'**il comprend un ou plusieurs illuminateur(s), qui sont adaptés pour éclairer ladite cassette (3), de manière à rendre l'analyse visuelle indépendante de la condition de la lumière ambiante ; ladite machine de dialyse comprenant une unité de commande (8) ayant un logiciel de gestion et de traitement de données configuré pour lire des images en provenance du capteur de vision (2) et pour les convertir en actions ou en alarmes en fonction de paramètres entrés précédemment ; ledit capteur de vision (2) étant capable de lire un code de la cassette et de transmettre les informations à l'unité de commande (8) qui est configurée pour vérifier la compatibilité entre la cassette et un type de traitement installé sur la machine et un kit jetable monté.

2. Machine de dialyse selon la revendication 1, **caractérisée en ce que** ledit illuminateur est un illuminateur à diode électroluminescente à lumière blanche.

3. Machine de dialyse selon la revendication 1 ou 2, **caractérisée en ce que** ledit capteur de vision (2) est adapté pour détecter des images dans un spectre de longueurs d'onde dans le domaine visible.

4. Machine de dialyse selon l'une des revendications précédentes, **caractérisée en ce que** ledit capteur de vision (2) présente une résolution de 640x480 pixels.
